# DEMANDE DE BREVET EUROPEEN

(11) **EP 1 222 916 A2**
(43) Date de publication de la demande: **17.07.2002**
(21) Numéro de dépôt: 02290573.1
(22) Date de dépôt: 19.06.1997
(51) Int. Cl.: A61K 7/48, A61K 7/06, A61K 31/17

(54) **Utilisation de dérivés amino-alcools à fonction urée comme hydratant dans des compositions cosmétiques ou dermatologiques**

(30) Priorité: 01.07.1996 FR 9608173
(62) Demande divisionnaire de: 97401418.5
(71) Demandeur: L'OREAL, 75008 Paris (FR)
(72) Inventeur: Philippe, Michel, 91320 Wissous (FR); Blaise, Christian, 94160 Saint-Mande (FR); Tuloup, R-my, 75014 Paris (FR)
(74) Mandataire: Dodin, Catherine

(57) **Abrégé**

L'invention concerne l'utilisation de dérivés amino alcools à fonction urée répondant à la formule suivante : 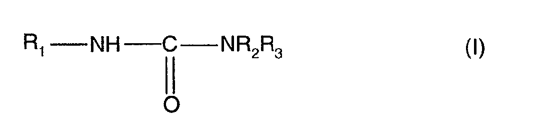 dans laquelle :
- R₁ représente un radical alcoyle, linéaire ou ramifié, saturé ou insaturé ayant de 8 à 22 atomes de carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone, comme agents hydratants dans des produits de soin pour la peau.

## Description

La présente invention concerne l'utilisation de dérivés amino alcools à fonction urée dans et pour la préparation de compositions cosmétiques ou dermatologiques en particulier leur utilisation comme agents hydratants dans des produits de soin pour la peau.

On connaît dans l'état de la technique un certain nombre de composés amino-alcools à fonction urée. Certains ont été notamment décrits dans le brevet US 3.135.790. et sont utilisés comme agents émulsifiants dans des formulations de peintures ou de laques ou comme intermédiaires de synthèse. D'autres tels que ceux décrits dans le brevet DE1125 905, notamment le N-méthyl-N-(2,3,4,5,6-penta-hydroxy-n-hexyl)-N'-n-octyl-urée, sont utilisés comme additifs pour textiles, agents de lavage, agents de réticulation ou comme émulsifiants. On utilise certains dérivés amino-alcools à fonction urée dans le brevet US 2.891.944 pour leurs propriétés insecticides. On a décrit également le 3-(3-octadécyluréido)-1,2-propanediol dans l'article J. Med. Chem. 31(4), 858-863, 1988, pour son activité sur certaines cellules cancéreuses.

La Demanderesse a découvert de manière surprenante que des dérivés amino-alcools à fonction urée particuliers dont on définira la structure ci-dessous présentaient des propriétés cosmétiques intéressantes, en particulier des propriétés hydratantes de la peau.
On a en effet constaté qu'en appliquant ces produits sur la peau, il est possible d'en diminuer la perte en eau et/ou d'augmenter la fixation d'eau dans le Stratum Corneum. Ces composés peuvent donc être utilisés comme agents hydratants, notamment de la peau chez l'humain.
Ils permettent de conserver ou de restaurer la souplesse de la peau, son élasticité, sa résistance au mouvement du corps et sa fonction de barrière à l'entrée des substances toxiques. Ils peuvent ainsi être utilisés dans des produits de soin pour les peaux sèches ou prédisposés à l'assèchement.
Ces composés présentent de façon inattendue une activité de barrière de perméabilité à l'eau sur la peau considérablement plus importante que leurs homologues carbamates ou amides connus en cosmétique pour leurs propriétés hydratantes.

L'objet principal de l'invention concerne donc l'utilisation de dérivés amino-alcools à fonction urée que l'on définira par la suite, comme agent hydratant dans une composition cosmétique de soin pour la peau ou pour la préparation d'une composition dermatologique pour le soin de la peau.

Un autre objet de l'invention concerne les compositions cosmétiques ou dermatologiques comprenant, dans un véhicule cosmétiquement ou dermatologiquement acceptable, de 0,001 à 15% en poids par rapport au poids total de la composition, d'au moins un dérivé aminoalcool à fonction urée de formule (I).

Les dérivés amino-alcools à fonction urée conformes à l'invention répondent à la formule générale (I) suivante: 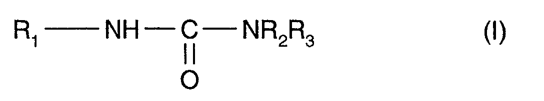 dans laquelle :
- R₁ représente un radical alcoyle, linéaire ou ramifié, saturé ou insaturé ayant de 8 à 22 atomes carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone.

De préférence, R₁ représente un radical alcoyle, saturé ou insaturé, linéaire ou ramifié, en C₁₀-C₁₈.
De préférence, R₂ représente un hydrogène ou le groupe méthyle.
De préférence, R₃ représente un radical -(CH₂)ₙ-(CHOH)ₘ-Z dans lequel n représente un nombre entier égal à 0 ou 1, m représente un nombre entier compris entre 0 et 5, et Z est un radical alcoyle monohydroxylé ou polyhydroxylé ayant de 1 à 4 atomes de carbone.
En particulier, Z est choisi parmi le groupe comprenant les radicaux suivants :

―CH₂OH ―CH₂-CH₂OH ―CH(CH₂OH)₂

―C(CH₂OH)₃

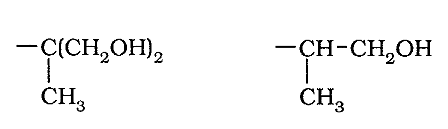

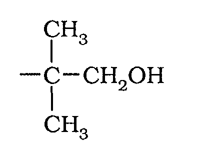

Les radicaux R₃ plus préférentiels sont choisis dans le groupe constitué par :
-CH₂-CHOH-CH₂OH; -CH₂-(CHOH)₄CH₂OH; -C-(CH₂OH)₃ et -C(CH₃)(CH₂OH)₂.

Parmi les composés préférés correspondant à la formule générale (I), on peut notamment citer :
- la N-dodécylaminocarbonyl-N-méthyl-D-glucamine ;
- la N-octylaminocarbonyl-N-méthyl-D-glucamine ;
- la 1-dodécyl-3-(2-hydroxy-1,1-bis-hydroxyméthyl-éthyl)urée ;
- la N-dodécylaminocarbonyl-D-glucamine ;
- la 1-(2,3-dihydroxypropyl)-3-dodécyl-urée ;
- la 1-dodécyl-3-(2-hydroxy-1-hydroxyméthyl-1-méthyl-éthyl)-urée ;
- la 1-(2-éthylhexyl)-3-(2-hydroxy-1,1-bis-hydroxyméthyl-éthyl)urée ;
- la N-(4Z)-octadéc-9-ènyl-aminocarbonyl-N-méthyl-D-glucamine.

Ces composés peuvent être obtenus par réaction, dans un solvant de préférence polaire, d'un aminoalcool de formule R₂-NH-R₃ dans laquelle R₂ et R₃ ont les mêmes significations que celles indiquées précédemment avec un composé imidazolo alkyl carboxamide de formule (II) :

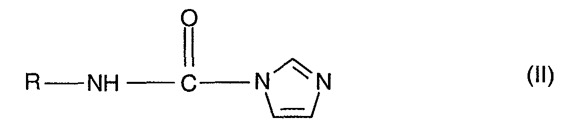

La réaction est généralement effectuée en atmosphère inerte en présence d'un solvant tel que le méthanol, à la température correspondant au reflux du solvant, de préférence entre 40 et 50°C.
Les composés imidazolo alkyl carboxamide de formule (II) sont obtenus selon des méthodes de synthèse connues, notamment dans la référence suivante : Angewandte Chemie, International Edition, Vol 1, n°7, 1962.

Les compositions cosmétiques ou dermatologiques selon l'invention sont caractérisées par le fait qu'elles contiennent dans un véhicule cosmétiquement ou dermatologiquement acceptable au moins un composé de formule (I) tel que défini ci-dessus à l'exception du composé N-méthyl-N-(2,3,4,5,6-penta-hydroxy-n-hexyl)-N'-n-octyl-urée.

Les compositions cosmétiques ou dermatologiques selon l'invention comprend de préférence 0,001 à 15% en poids de composé de formule (I) par rapport au poids total de la composition.

Le véhicule cosmétiquement acceptable utilisé dans les compositions de l'invention est choisi parmi l'eau; les solvants organiques compatibles avec une application cutanée ou capillaire tels que l'acétone, l'isopropanol, l'éthanol; les triglycérides d'acides gras à 6-24 atomes de carbone, les éthers de glycol, les esters de polyalkylèneglycols et les silicones volatiles ou leurs mélanges.

Les compositions peuvent se présenter sous forme de lotion aqueuse ou hydroalcoolique, monophasique ou polyphasique, de gel monophasique ou polyphasique, d'émulsion, de crème, de dispersion vésiculaire de lipides ioniques ou non ioniques, lesdites vésicules pouvant alors servir en tant qu'agent d'encapsulation pour des ingrédients actifs lipophiles ou hydrophiles, de mousse, de spray.

Les compositions pour le soin de la peau selon l'invention peuvent se présenter sous forme de lotion, de gel, d'émulsion, de crème ou de mousse à appliquer sur la peau.

Les compositions cosmétiques ou dermatologiques peuvent par ailleurs contenir des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les colorants, les parfums, les conservateurs, les agents propulseurs.
Plus précisément, comme corps gras, on peut utiliser une huile ou une cire ou leur mélange, des acides gras, des alcools gras, des esters d'acides gras tels que les triglycérides d'acide gras en C6 à C18, de la vaseline, de la paraffine, de la lanoline, de la lanoline hydrogénée ou acétylée.
Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone et les isoparaffines.
Parmi les cires, on peut citer les cires animales, végétales, minérales ou de synthèse, et notamment les cires d'abeilles, de Candelila, les ozokérites, les cires microcristallines ainsi que les cires et résines de silicone.
Parmi les solvants organiques usuellement utilisés dans les compositions cosmétiques, on peut citer plus précisément les mono-alcools ou polyalcools inférieurs en C₁ à C₆ comme l'éthanol, l'isopropanol, l'éthylèneglycol, le diéthylènegycol, le propylèneglycol, le glycérol.
Les agents épaississants peuvent être choisis notamment parmi l'alginate de sodium, la gomme arabique, les dérivés cellulosiques tels que la méthylcellulose, l'hydroxy méthylcellulose, l'hydroxyéthylcellulose, l'hydroxypropylcellùlose, l'hydroxypropylméthyl cellulose, la gomme de guar ou ses dérivés, la gomme de xanthane, les scléroglucanes, les acides polyacryliques réticulés.
Comme agents tensio-actifs et comme polymères, on peut utiliser tous ceux bien connus de l'état de la technique notamment pour leur utilisation dans des compositions capillaires.

Les compositions peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans STANDISH & WATKINS, J. Mol. Biol., 13,238 (1965) ou dans les brevets FR-A-2.315.991 et FR-A-2.416.008 de la demanderesse. Les différents types de procédés de préparation sont décrits dans "Les liposomes en biologie cellulaire et pharmacologie", Edition INSERM/John Libery Eurotext, 1987, pages 6 à 18.

Le pH des compositions selon l'invention est généralement compris entre 4 et 8 et de préférence entre 5 et 7.

L'invention est illustrée plus en détail dans les exemples suivants.

### Exemple 1 : Synthèse de la N-dodécylaminocarbonyl-N-méthyl-D-glucamine

### Première étape : Synthèse du 1-N-dodécylaminocarbonyl-imidazole de formule (II) (R₁ = C₁₂H₂₅)

Dans un réacteur, on dissout 49 g de N,N'-carbonyldiimidazole 700 cm³ de diméthylformamide anhydre et on maintient sous atmosphère inerte. On ajoute, de manière à maintenir la température à une valeur inférieure à 30°C, 55g de laurylamine. On maintient sous agitation à température ambiante pendant 8 heures. On verse le mélange réactionnel résultant sur 2 litres d'eau et on filtre le précipité obtenu. Après séchage, on obtient 75 g (rendement 90 %) de 1-N-dodécylaminocarbonyl-imidazole sous forme de poudre blanche.

### Deuxième étape : Synthèse de la N-dodécylaminocarbonyl-N-méthyl-D-glucamine de formule (I) (R₁ = C₁₂H₂₅, R₂ = CH₃, R₃ = -CH₂-(CHOH)₄-CH₂OH)

On met en suspension, sous atmosphère inerte, 42g de 1-N-dodécylaminocarbonyl-imidazole et 32 g de N-méthyl-D-glucamine dans 400 cm³ de méthanol. On maintient le mélange réactionnel au reflux sous agitation pendant 4 heures. On refroidit par un bain de glace puis on filtre le précipité. On le lave par 500 cm³ d'eau puis on le sèche.
On obtient 49g (rendement 80 %) de N-dodécylaminocarbonyl-N-méthyl-D-glucamine sous forme de poudre blanche.

### Analyse élémentaire :

| | C | H | N | O |
|---|---|---|---|---|
| Calculée (%) | 59,09 | 10,41 | 6,89 | 23,61 |
| Trouvée (%) | 58,83 | 10,45 | 6,96 | 23,37 |

Spectre RMN 1H 200 MHz conforme
Point de fusion : 116,8°C (FP 89)

### Exemple 2 : Synthèse de la 1-dodécyl-3-(2-hydroxy-1,1-bis-hydroxyméthyléthyl)urée de formule (I) (R₁=C₁₂H₂₅, R₂= H, R₃ = -C(CH₂OH)₃)

On procède selon le même procédé décrit dans l'exemple 1 à partir de 5,2 g de trihydroxyméthylaminométhane de formule R₂-NH-R₃ correspondant où R₂ désigne H et R₃ désigne -C(CH₂OH)₃ et 8 g de 1-N-dodécylaminocarbonyl-imidazole de formule (II) correspondant où R₁ désigne C₁₂H₂₅ dans 100 cm³ de méthanol.

### Analyse élémentaire :

| | C | H | N | O |
|---|---|---|---|---|
| Calculée (%) | 61,41 | 10,91 | 8,43 | 19,25 |
| Trouvée (%) | 61,40 | 11,08 | 8,29 | 19,12 |

Spectre RMN 1H 200 MHz conforme
Point de fusion : 132,4°C (FP 89)

### Exemple 3 : Synthèse de la N-dodécylaminocarbonyl- D-glucamine de formule (I) (R₁ = C₁₂H₂₅, R₂ = H, R₃ = -CH₂-(CHOH)₄-CH₂OH)

On procède selon le même procédé décrit dans l'exemple 1 à partir de 6,1 g de D-glucamine de formule R₂-NH-R₃ correspondant où R₂ désigne H et R₃ désigne -CH₂-(CHOH)₄-CH₂OH) et 7g de 1-N-dodécylaminocarbonyl-imidazole de formule (II) correspondant où R₁ désigne C₁₂H₂₅ dans 100 ml de méthanol.

### Analyse élémentaire :

| | C | H | N | O |
|---|---|---|---|---|
| Calculée (%) | 58,14 | 10,27 | 7,14 | 24,46 |
| Trouvée (%) | 58,09 | 10,21 | 7,18 | 24,73 |

Spectre RMN 1H 200 MHz conforme
Point de fusion : 161,5°C (FP 89)

### Exemple 4 : Synthèse de la 1-(2,3-dihydroxypropyl)-3-dodécyl-urée de formule (I) (R₁ = C₁₂H₂₅, R₂ = H, R₃ = -CH₂-CHOH-CH₂OH)

On procède selon le même procédé décrit dans l'exemple 1 à partir 2,3 g de 3-amino-1,2-propanediol de formule R₂-NH-R₃ correspondant où R₂ désigne H et R₃ désigne -CH₂-CHOH-CH₂OH et de 7 g de 1-N-dodécyaminocarbonyl-imidazole de formule (II) correspondant où R₁ désigne C₁₂H₂₅ dans 100 cm³ de méthanol.

### Analyse élémentaire :

| | C | H | N | O |
|---|---|---|---|---|
| Calculée (%) | 63,54 | 11,33 | 9,26 | 15,87 |
| Trouvée (%) | 63,51 | 11,45 | 9,14 | 16,09 |

Spectre RMN 1H 200 MHz conforme
Point de fusion : 121,5°C (FP 89)

### Exemple 5 : Synthèse de la 1-dodécyl-(2-hydroxy-1-hydroxyméthyl-1-méthyléthyl)-urée de formule (I) (R₁ = C₁₂H₂₅, R₂ = H, R₃ = -C(CH₃)(CH₂OH)₂)

On procède selon le même procédé décrit dans l'exemple 1 à partir de 3,5 g de 2-amino-2-méthyl-1,3-propanediol de formule R₂-NH-R₃ correspondant où R₂ désigne H et R₃ désigne -C(CH₃)(CH₂OH)₂
et de 7 g de 1-N-dodécylaminocarbonyl-imidazole de formule (II) correspondant où R₁ désigne C₁₂H₂₅ dans 100 cm³ de méthanol.

### Analyse élémentaire :

| | C | H | N | O |
|---|---|---|---|---|
| Calculée (%) | 64,52 | 11,47 | 8,85 | 15,17 |
| Trouvée (%) | 64,52 | 11,43 | 8,84 | 15,00 |

Spectre RMN 1H 200 MHz conforme
Point de fusion : 60,1 °C (FP 89)

### Exemple 6 : Synthèse de la 1-(2-éthylhexyl)-3-(2-hydroxy-1,1-bis-hydroxyméthyl éthyl)urée de formule (I) (R₁ = C₄H₇-CH(C₂H₅)-CH₂, R₂ = H, R₃ = -C(CH₂OH)₃)

On procède selon le même procédé décrit dans l'exemple 1 à partir de 14 g de trihydroxy méthylaminométhane de formule R₂-NH-R₃ correspondant où R₂ désigne H et R₃ désigne -C(CH₂OH)₃ et de 25 g de 1-N-2-éthylhexyl-aminocarbonyl-imidazole de formule (II) correspondant où R₁ désigne C₄H₇-CH(C₂H₅)-CH₂- dans 200 cm³ de méthanol.

### Analyse élémentaire :

| | C | H | N | O |
|---|---|---|---|---|
| Calculée (%) | 56,50 | 10,21 | 10,14 | 23,16 |
| Trouvée (%) | 56,56 | 10,35 | 10,23 | 23,38 |

Spectre RMN 1H 200 MHz conforme
Point de fusion : 155,8°C (FP 89)

### Exemple 7 : Synthèse de la N-(4Z)-octadéc-9-ènyl-aminocarbonyl-N-méthyl-D-glucamine de formule (I) (R₁ = C₈H₁₇-CH=CH-C₈H₁₆, R₂ = CH₃, R₃ = -CH₂-(CHOH)₄-CH₂OH)

On procède selon le même procédé décrit dans l'exemple 1 à partir de N-méthyl-D-glucamine de formule R₂-NH-R₃ correspondant où R₂ désigne CH₃ et R₃ désigne -CH₂-(CHOH)₄-CH₂OH et 48 g de 1-N-octadéc-9-ènyl-aminocarbonyl-imidazole de formule (II) correspondant où R₁ désigne C₈H₁₇-CH=CH-C₈H₁₆ dans 300 cm³ de méthanol.

### Analyse élémentaire :

| | C | H | N | O |
|---|---|---|---|---|
| Calculée (%) | 63,90 | 10,72 | 5,73 | 19,64 |
| Trouvée (%) | 64,07 | 10,75 | 5,85 | 19,90 |

Spectre RMN 1H 200 MHz conforme
Point de fusion : 116,2°C (FP 89)

### Exemple 8 : Synthèse de la N-octylaminocarbonyl-N-méthyl-D-glucamine de formule (I) (R₁ = C₈H₁₇, R₂ = CH₃, , R₃ = -CH₂-(CHOH)₄-CH₂OH)

### 1/ Synthèse du -N-octylaminocarbonyl-imidazole

On dissout 80 g de N,N'-carbonyldiimidazole dans 900 cm3 de diméthylformamide anhydre et on maintient sous atmosphère inerte. On ajoute 63 g d'octyl en maintenant la température en dessous de 30°C. On maintient sous agitation à température ambiante pendant 4 heures. On verse sur 3 litres pour extraire par 4 fois 400 cm3 d'acétate d'éthyle. On sèche, on filtre et on évapore jusqu'à obtenir 84 g de produit sous forme d'une huile pâteuse (Rendement : 86%). Le Spectre RMN 1H 200 MHz est conforme.

### 2/ Synthèse de la N-octylaminocarbonyl-N-méthyl-D-glucamine

On met en suspension, sous atmosphère inerte, 47g de 1-N-octylaminocarbonyl-imidazole et 42 g de N-méthyl-D-glucamine dans 300 cm³ de méthanol. On maintient le mélange réactionnel au reflux sous agitation pendant 3 heures. On refroidit par un bain de glace puis on filtre le précipité. On le recristallise dans 300 cm³ d'éthanol.
On obtient 40 g (rendement 55%) de N-octylaminocarbonyl-N-méthyl-D-glucamine sous forme de poudre blanche.
Spectre RMN 1H 200 MHz conforme
Spectre de masse conforme
Point de fusion : 111,4°C (FP 89)

### Analyse élémentaire :

| | C | H | N | O |
|---|---|---|---|---|
| Calculée (%) | 54,84 | 9,78 | 7,99 | 27,39 |
| Trouvée (%) | 53,58 | 9,71 | 8,25 | 28,34 |

### Exemple 9 : Exemple comparatif

### Mesure de la perte insensible en eau (PIE)

Cette mesure s'effectue à l'aide d'un évaporimètre (Servomed) qui détermine quantitativement l'évaporation d'eau, c'est-à-dire un transport d'eau par diffusion, à partir d'un échantillon de Stratum Corneum obturant une capsule cylindrique contenant de l'eau, le tout étant placé dans une chambre à température et humidité relatives contrôlées.
Des capteurs permettent de mesurer la pression partielle de vapeur d'eau en deux points situés à des distances différentes de l'échantillon.
On détermine ainsi le gradient de pression partielle de vapeur d'eau entre les deux points, et donc le taux d'évaporation conformément à la loi de Fick.

Cette mesure est effectuée pour :
a) la N-dodécylaminocarbonyl-N-méthyl-D-glucamine (composé de l'exemple 1) ;
b) la N-dodécanoyl-N-méthyl-D-glucamine, homologue amide du composé de l'exemple 1, connu pour être un agent hydratant que l'on nommera référence A ;
c) la N-dodécyloxycarbonyl-N-méthyl-D-glucamine, homologue carbamate du composé de l'exemple 1, connu pour être un agent hydratant que l'on nommera référence B.

Les composés sont préparés à raison de 1% dans un mélange de dichlorométhane/méthanol (2/1 v/v), puis appliqués sur des morceaux de Stratum Corneum délipidé.
On mesure, 20 heures après l'application, la perte insensible en eau (PIE) pour chaque composé.
On obtient les résultats suivants

| Composé | exemple 1 | référence A | référence B |
|---|---|---|---|
| P.I.E. | -21 ±2 | - 11 ±3 | - 9 ± 2 |

On constate donc que l'application du composé selon l'invention permet de réduire de façon plus importante l'évaporation de l'eau contenue dans le Stratum Corneum par rapport à ses homologues amide et carbamate.

### Exemple 10 : Crème de jour pour le visage

On prépare la composition suivante:
. alcool cétylique 2,5%
. tristéarate de sorbitan 0,9%
. stéarate de polyoxyde d'éthylène (polyoxyéthyléné 40 fois) 2%
. stéarate de glycéryle 3%
. myristate de myristyle 2%
. polyisobutène hydrogéné 2,5%
. palmitate d'octyle 4%
. polydiméthylsiloxane (10 cm².s) 5%
. huile de noyaux d'abricot 5,715%
. N-dodécylaminocarbonyl-N-méthyl-D-glucamine 0,5%
. conservateurs 0,5%
. eau qsp 100%

On obtient une crème de jour se présentant sous forme d'une émulsion permettant de bien couvrir et protéger la peau, particulièrement adaptée pour les peaux normales et sèches.

### Exemple 11 : Crème de nuit

On prépare la formulation suivante:
. mélange de mono- et di-stéarate de glycéryle et de stéarate de polyoxyde d'éthylène 2%
. N-dodécylaminocarbonyl-N-méthyl-D-glucamine 3%
. huile de noyaux d'abricot 17%
. cyclopentadiméthylsiloxane 1,5%
. Carbomer (vendu sous la marque CARBOPOL) 0,75%
. conservateurs 0,5%
. triéthanolamine 0,75%
. eau qsp 100%

On obtient une crème de nuit, se présentant sous forme d'une émulsion épaisse, brillante et très douce à appliquer, qui se révèle nourrissante et hydratante pour la peau, particulièrement pour les peaux sèches.

## Revendications

1. Utilisation cosmétique d'au moins un dérivé aminoalcool à fonction urée de formule (I) : 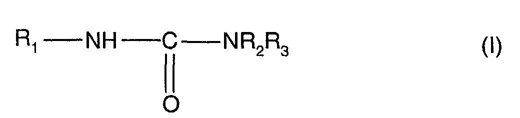 dans laquelle :
- R₁ représente un radical alcoyle, linéaire ou ramifié, saturé ou insaturé ayant de 8 à 22 atomes carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone,
comme agent hydratant dans une composition cosmétique de soin pour la peau.

2. Utilisation d'au moins un dérivé aminoalcool à fonction urée de formule (I) : 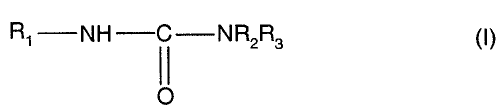 dans laquelle :
- R₁ représente un radical alcoyle, linéaire ou ramifié, saturé ou insaturé ayant de 8 à 22 atomes carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone,
pour la préparation d'une composition dermatologique pour le soin de la peau.

3. Utilisation selon l'une des revendications précédentes, dans des produits de soin pour les peaux sèches ou prédisposées à l'assèchement.

4. Utilisation selon l'une des revendications précédentes, dans laquelle :
- R₁ représente un radical alcoyle, linéaire ou ramifié, saturé ou insaturé en C₁₀-C₁₈,
- R₂ représente un hydrogène ou le groupe méthyle et/ou
- R₃ représente un radical -(CH₂)ₙ-(CHOH)ₘ-Z dans lequel n représente un nombre entier égal à 0 ou 1, m représente un nombre entier compris entre 0 et 5, et Z est un radical alcoyle monohydroxylé ou polyhydroxylé ayant de 1 à 4 atomes de carbone.

5. Utilisation selon l'une des revendications précédentes, dans laquelle R₃ est choisi dans le groupe constitué par :
-CH₂-CHOH-CH₂OH; -CH₂-(CHOH)₄CH₂OH; -C-(CH₂OH)₃ et -C(CH₃)(CH₂OH)₂.

6. Utilisation selon l'une des revendications précédentes, dans laquelle les dérivés de formule (I) sont choisis parmi :
- la N-dodécylaminocarbonyl-N-méthyl-D-glucamine ;
- la N-octylaminocarbonyl-N-méthylglucamine ;
- la 1-dodécyl-3-(2-hydroxy-1,1-bis-hydroxyméthyl-éthyl)urée ;
- la N-dodécylaminocarbonyl-D-glucamine ;
- la 1-(2,3-dihydroxypropyl)-3-dodécyl-urée ;
- la 1-dodécyl-3-(2-hydroxy-1-hydroxyméthyl-1-méthyl-éthyl)-urée ;
- la 1-(2-éthylhexyl)-3-(2-hydroxy-1,1-bis-hydroxyméthyl-éthyl)urée ;
- la N-(4Z)-octadéc-9-ènyl-aminocarbonyl-N-méthyl-D-glucamine.

7. Utilisation selon l'une des revendications précédentes, dans laquelle le dérivé de formule (I) est présent en une quantité de 0,001 à 15% en poids par rapport au poids total de la composition.

8. Composition cosmétique ou dermatologique comprenant, dans un véhicule cosmétiquement ou dermatologiquement acceptable, de 0,001 à 15% en poids par rapport au poids total de la composition, d'au moins un dérivé aminoalcool à fonction urée de formule (I) : 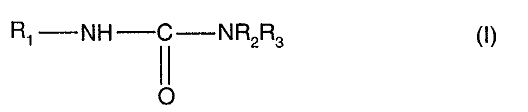 dans laquelle :
- R₁ représente un radical alcoyle, linéaire ou ramifié, saturé ou insaturé ayant de 8 à 22 atomes carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone ; à l'exception du composé N-méthyl-N-(2,3,4,5,6-penta-hydroxy-n-hexyl)-N'-n-octyl-urée.

9. Composition selon la revendication 8, dans laquelle :
- R₁ représente un radical alcoyle, linéaire ou ramifié, saturé ou insaturé en C₁₀-C₁₈,
- R₂ représente un hydrogène ou le groupe méthyle et/ou
- R₃ représente un radical -(CH₂)ₙ-(CHOH)ₘ-Z dans lequel n représente un nombre entier égal à 0 ou 1, m représente un nombre entier compris entre 0 et 5, et Z est un radical alcoyle monohydroxylé ou polyhydroxylé ayant de 1 à 4 atomes de carbone.

10. Composition selon l'une des revendications 8 à 9, dans laquelle R₃ est choisi dans le groupe constitué par :
-CH₂-CHOH-CH₂OH; -CH₂-(CHOH)₄CH₂OH; -C-(CH₂OH)₃ et -C(CH₃)(CH₂OH)₂.

11. Composition selon l'une des revendications 8 à 10, dans laquelle les dérivés de formule (I) sont choisis parmi :
- la N-dodécylaminocarbonyl-N-méthyl-D-glucamine ;
- la 1-dodécyl-3-(2-hydroxy-1,1-bis-hydroxyméthyl-éthyl)urée ;
- la N-dodécylaminocarbonyl-D-glucamine ;
- la 1-(2,3-dihydroxypropyl)-3-dodécyl-urée ;
- la 1-dodécyl-3-(2-hydroxy-1-hydroxyméthyl-1-méthyl-éthyl)-urée ;
- la 1-(2-éthylhexyl)-3-(2-hydroxy-1,1-bis-hydroxyméthyl-éthyl)urée ;
- la N-(4Z)-octadéc-9-ènyl-aminocarbonyl-N-méthyl-D-glucamine.

12. Composition selon l'une quelconque des revendications 8 à 11, **caractérisée par le fait qu'**elle se présente sous forme de lotion aqueuse ou hydroalcoolique, monophasique ou polyphasique, de gel monophasique ou polyphasique, d'émulsion, de crème, de dispersion vésiculaire de lipides ioniques ou non ioniques, lesdites vésicules pouvant alors servir en tant qu'agent d'encapsulation pour des ingrédients actifs lipophiles ou hydrophiles, de mousse, de spray.
